Europäisches Patentamt

European Patent Office

Office européen des brevets

(11) Publication number: **0 361 348**
**A1**

## (12) EUROPEAN PATENT APPLICATION

(21) Application number: 89117635.6

(22) Date of filing: 25.09.89

(51) Int. Cl.⁵: **A61K 47/00** , **A61K 37/02** ,
**//(A61K37/02,31:54)**

Claims for the following Contracting States: ES
+ GR.

(30) Priority: 26.09.88 JP 240447/88

(43) Date of publication of application:
**04.04.90 Bulletin 90/14**

(84) Designated Contracting States:
**AT BE CH DE ES FR GB GR IT LI LU NL SE**

(71) Applicant: **NIPPON HYPOX LABORATORIES
INCORPORATED**
**1759, Matsugaya Hachioji-shi**
**Tokyo(JP)**

(72) Inventor: **Satoh, Toshio**
**57-3, Nagao Jyoroku-cho**
**Tokushima-shi Tokushima-ken(JP)**
Inventor: **Matsumoto, Hitoshi**
**125-22, Shimofukuman Hachiman-cho**
**Tokushima-shi Tokushima-ken(JP)**

(74) Representative: **Hansen, Bernd, Dr.rer.nat. et
al**
**Hoffmann, Eitle & Partner Patentanwälte**
**Arabellastrasse 4 Postfach 81 04 20**
**D-8000 München 81(DE)**

(54) Non-steroidal pharmaceutical composition and process for producing the same.

(57) The present invention relates to a non-steroidal anti-inflammatory agent composition comprising Piroxicam and a natural albumin. This composition is characterized by being easily absorbed and alleviating the side effects (e.g. peptic ulcer formation) caused by Piroxicam.

EP 0 361 348 A1

## Non-steroidal Pharmaceutical Composition and Process for Producing the Same

BACKGROUND OF THE INVENTION

(1) Field of the Invention

The present invention relates to a non-steroidal pharmaceutical composition and a process for producing the composition.

(2) Description of the Prior Art

Non-steroidal anti-inflammatory agents show a strong anti-inflammatory action to inflammation-affected parts, but they are known to give serious side effects to the central nervous system and the digestive system, in particular, hemorrhage and ulcer formation in the digestive system. In order to alleviate these side effects, various pharmaceutical compositions have been studied and reported. For example, there is known a pharmaceutical composition comprising a non-steroidal anti-inflammatory agent or a salt thereof and 0.2-50 parts by weight of salicyclic acid or an alkali metal salicylate based on 1 part by weight of the former [Japanese Patent Application Kokai (Laid-Open) No. 82715/1976]. Further, it is known to include Indometacin in a biodegradable carrier selected from collagen and gelatin (Japanese Patent Application Kokai (Laid-Open) No. 84213/1985), or to include Indometacin in cyclodextrin to enable the sustained release of Indometacin [Japanese Patent Application Kokai (Laid-Open) Nos. 62313/1979 and 117018/1979]. Furthermore, there is disclosed in Japanese Patent Application Kokai (Laid-Open) No. 110334/1979 a method wherein Indometacin is mixed with a benzodiazepine compound to thereby reduce the administration amount of Indometacin and to inhibit the expression of its serious side effect.

Though these conventional methods are superior in sustained release of the drug, they are poor in absorption of the drug into human body, take a long time before the drug exhibits an anti-inflammatory action, and are insufficient in the alleviation of the side effect of the drug on the digestive system.

Piroxicam is a relatively new non-steroidal anti-inflammatory agent but, like other non-steroidal anti-inflammatory agents, this agent also has side effects such as peptic ulcer formation and the like. Therefore, alleviation of Piroxicam's side effects has been studied according to the above mentioned conventional techniques. However, no method has been found yet in which the side effects of Piroxicam can be alleviated without reducing its absorbability into human body.

SUMMARY OF THE INVENTION

Hence, objects of the present invention are to provide a non-steroidal anti-inflammatory agent composition comprising Piroxicam as an active ingredient, which has excellent absorbability and gives reduced gastroenteric disorder, as well as a process for producing the composition.

The present invention has been made in order to achieve the above objects. The present invention relates to a non-steroidal anti-inflammatory agent composition comprising Piroxicam and a natural albumin.

The present invention relates also to a process for producing a non-steroidal anti-inflammatory agent composition, which process comprises stirring Piroxicam and a natural albumin at a high speed in the presence of an aqueous solvent and then removing the aqueous solvent to obtain a non-steroidal anti-inflammatory agent composition comprising Piroxicam and a natural albumin.

Japanese Patent Application Kokai (Laid-Open) No. 216126/1983 describes the use of human serum albumin in order that a drug with no or very low solubility in water becomes water-soluble. The solubilization method described in the above patent document, however, is the application of the conventionally known method for measurement of the protein binding ratio of a drug; in this method, a drug can be solubilized only in a very small amount of about 0.01-0.8% based on the serum albumin; accordingly, the method is exceptionally useful only when a drug is used at a very low dosage as in the case of Prostaglandin shown in the above patent document.

In contrast, the non-steroidal anti-inflammatory agent composition of the present invention can contain an anti-inflammmatory agent (Piroxicam) in a high concentration as described in detail below and accordingly is essentially different from the technique described in the above patent document.

2

As the albumin used in the non-steroidal anti-inflammatory agent composition of the present invention, there can be mentioned, for example, egg albumin, serum albumin, plakalbumin, α-lactalbumin, leucosin, phaselin and Revmerine. Of these, egg albumin is especially preferred. The use of a human-derived albumin is preferred when antigenicity is concerned.

The non-steroidal anti-inflammatory agent composition of the present invention can be produced by stirring Piroxicam and a natural albumin at a high speed in the presence of an aqueous solvent and then removing the aqueous solvent. The composition can also be produced by dissolving Piroxicam in a fatty oil and/or a fatty acid, adding the resulting Piroxicam solution to a suspension of a natural albumin in an aqueous solvent and stirring them at a high speed, and then removing the aqueous solvent.

In the above production, the concentration of natural albumin in suspension is not particularly restricted but is at least 1% (w/v), preferably 5-25% (w/v). The proportion of Piroxicam to natural albumin can be varied in a wide range, but Piroxicam is generally used in an amount of preferably 5-100 parts by weight based on 100 parts by weight of natural albumin.

The aqueous solvent can be any as long as it can dissolve the natural albumin without denaturing it, but water is most preferred. Part of water can be replaced by a hydrophilic solvent such as methanol, ethanol, acetone, ethyl acetate or the like. It is also possible to use, together with water and the hydrophilic solvent, a hydrophobic solvent such as chloroform, dichloroethane or the like.

The reaction temperature and the reaction time are not critical, but it is generally advantageous that the reaction be effected at a temperature at which the natural albumin causes no thermal denaturation, preferably at room temperature for about 1-30 hours with stirring Piroxicam and the natural albumin at a high speed, preferably at about 5,000-40,000 rpm.

In dissolving Piroxicam in a fatty oil and/or a fatty acid and then adding the resulting Piroxicam solution to an aqueous suspension of a natural albumin, the dissolution of Piroxicam in a fatty oil and/or a fatty acid is effected preferably using an organic solvent. As the organic solvent, there can be mentioned acetone, methanol, ethanol, chloroform, ethyl acetate, ether, dichloromethane, THF, etc.

A weight ratio of a fatty oil and/or a fatty acid to a mixture of Piroxicam and a natural albumin is preferably 1/9 -7/3, more preferably 2/8 - 6/4, most preferably 2.5/7.5 -5.5/4.5.

In the production of the composition of the present invention, it is unnecessary to use a solubilizing agent such as carboxymethyl cellulose, gum arabic, dimethylformamide, dimethyl sulfoxide or the like, or to use a surfactant which is employed in the production of albumin spherules; accordingly, there is no fear of the side effect caused by the inclusion of a solubilizing agent or a surfactant in the non-steroidal anti-inflammatory agent composition. As the fatty oil, there can be used olive oil, corn oil, coconut oil, soybean oil, sesame oil, etc. As the fatty acid, there can be used straight chain fatty acids having 8-30 carbon atoms.

Piroxicam and the fatty oil and/or the fatty acid are stirred usually at about 15-65° C for about 30-120 minutes. In this case, it is possible to distil off, if necessary, part of the organic solvent by blowing of nitrogen gas or under reduced pressure.

The concentration of Piroxicam in organic solvent is not particularly restricted, but Piroxicam can be used in a concentration of at least 0.5% (w/v), preferably 1-10% (w/v). The proportion of Piroxicam solution to fatty oil and/or fatty acid is not particularly restricted, either, but the Pyroxicam solution can be used in a proportion of not more than 100% (v/v), preferably 1-50% (v/v).

The uniform suspension obtained by the above mixing and high speed stirring is subjected to solvent removal by means of air drying, vacuum drying, drum drying, spray drying, freeze-drying or the like, whereby an intended non-steroidal anti-inflammatory agent composition of powder form can be obtained.

The excellent anti-inflammatory action and reduced side effect of the non-steroidal anti-inflammatory agent composition of the present invention have been proven by Examples which are given below.

Example 1

Production of non-steroidal anti-inflammatory agent composition No. 1 of the present invention

1 g of Piroxicam and 4.3 g of olive oil were dissolved in 25 ml of acetone to obtain a Piroxicam solution in acetone. Separately, 9 g of egg albumin was dissolved in 180 ml of purified water to obtain an aqueous albumin solution. The weight ratio of Piroxicam to the egg albumin was 1/9, and the weight ratio of the olive oil to the mixture of Piroxicam and the egg albumin was 3/7.

The two solutions were mixed and stirred, with ice cooling, for 2 hours at 25,000 rpm using a biomixer (BM-4 Model manufactured by Nihon Seiki Seisakusho). Then, the solvent was removed by distillation, and the residue was freeze dried by a conventional method to obtain 13.6 g of an anti-inflammatory agent composition No. 1 comprising Piroxicam, egg albumin and olive oil. The composition was a white powder and had very low solubility in water, methanol and ethanol.

Example 2

Production of non-steroidal anti-inflammatory agent composition No. 2 of the present invention

The procedure of Example 1 was repeated except that the weight ratio of the olive oil to the mixture of Piroxicam and the egg albumin was changed to 1/9, to obtain an anti-inflammatory agent composition No. 2.

Example 3

Production of non-steroidal anti-inflammatory agent composition No. 3 of the present invention

The procedure of Example 1 was repeated except that the weight ratio of the olive oil to the mixture of Piroxicam and the egg albumin was changed to 5/5, to obtain an anti-inflammatory agent composition No. 3.

Example 4

Production of non-steroidal anti-inflammatory agent composition No. 4 of the present invention

The procedure of Example 1 was repeated except that the olive oil was not used, to obtain an anti-inflammatory agent composition No. 4.

Pharmacological Tests

(1) Anti-inflammatory action of non-steroidal anti-inflammatory agent compositions No. 1-4 of the present invention

[Test procedure]

As test animals, there were used male SD rats each weighing about 200 g. As a test sample, there was used a suspension of each of the anti-inflammatory agent compositions No. 1-4 obtained in Examples 1-4 above, in a gum arabic solution (the concentration of Piroxicam in the suspension was 1%). This test sample was orally administered to each rat in an amount of 1 mg/kg in terms of Piroxicam. One hour after the administration of the test sample, an inflammatory agent (0.1 ml of a physiological saline solution containing 1% of carrageenin) was subcutaneously injected to each rat at the sole. Then, the sole volume was measured with the lapse of time using a plethysmometer, and swelling percent was calculated from the following formula.

Swelling percent (%) = $[(Ps - Po)/Po] \times 100$
where
$Po$ = sole volume before injection of carrageenin
$Ps$ = sole volume at a certain time after the injection of carrageenin
For comparison, Piroxicam alone was orally administered in an amount of 1 mg/kg, in the same manner as above, and the change of swelling percent with time was examined.

[Results]

4

The results are shown in Table 1.

Table 1

Change of swelling percent with time

| | | Swelling percent | | | | | |
|---|---|---|---|---|---|---|---|
| | | 0.5 hour | 1 hour | 1.5 hours | 2 hours | 3 hours | 4 hours |
| Control | Mean | 26.63 | 41.51 | 47.97 | 58.47 | 62.73 | 55.72 |
| | S.E. | 1.32 | 4.65 | 3.55 | 3.28 | 4.03 | 3.87 |
| Piroxicam alone | Mean | 26.79 | 39.51 | 46.71 | 45.72 | 43.77 | 44.33 |
| | S.E. | 2.04 | 2.78 | 3.24 | 3.68 | 3.60 | 3.54 |
| Inflammatory agent composition No. 1 | Mean | 15.49 | 19.46 | 23.98 | 24.38 | 29.45 | 30.40 |
| | S.E. | 1.82 | 3.49 | 3.34 | 3.36 | 3.73 | 4.13 |
| Inflammatory agent composition No. 2 | Mean | 24.41 | 29.47 | 31.20 | 37.37 | 41.76 | 43.98 |
| | S.E. | 3.96 | 4.64 | 3.25 | 3.61 | 3.40 | 4.22 |
| Inflammatory agent composition No. 3 | Mean | 17.48 | 25.83 | 25.61 | 28.57 | 27.81 | 27.26 |
| | S.E. | 3.10 | 4.31 | 2.15 | 2.69 | 2.42 | 3.34 |
| Inflammatory agent composition No. 4 | Mean | 22.31 | 26.06 | 35.78 | 34.77 | 38.88 | 41.01 |
| | S.E. | 3.13 | 2.56 | 3.34 | 2.78 | 2.44 | 3.08 |

Mean indicates an average value (n=5).

S.E. indicates a standard error.

Inflammatory agent composition

No. 1 ... Piroxicam (P)/albumin (A) = 1/9

Olive oil (O)/(P + A) = 3/7

No. 2 ... P/A = 1/9, O/(P + A) = 1/9

No. 3 ... P/A = 1/9, O/(P + A) = 5/5

No. 4 ... P/A = 1/9  No olive oil used.

It was confirmed from Table 1 that Piroxicam alone and the Piroxicam-albumin compositions No. 1-4 both showed a significant anti-inflammatory action but the Piroxicam-albumin compositions No. 1-4 were superior in the action. Especially the Piroxicam-albumin compositions No. 1 and 3 wherein the weight ratio of the olive oil to the mixture of Piroxicam and the albumin are 3/7 and 5/5, respectively, showed the best anti-inflammatory action.

(2) Inhibitory action of non-steroidal anti-inflammatory agent composition No. 1 of the present invention for peptic ulcer formation

[Test procedure]

As test animals, there were used male SD rats each weighing about 200 g. As a test sample, there was used a suspension of the anti-inflammatory agent composition No. 1 obtained in Example 1 above, in a gum arabic solution (the concentration of Piroxicam in the suspension was 1%). This test sample was orally administered to each rat which had been fasted for 24 hours, in an amount of 50 mg/kg in terms of Piroxicam. 8 hours after the administration, the stomach of each rat was removed and the length of the ulcer formed therein was measured using a stereoscopic microscope. For comparison, Piroxicam alone was orally administered in an amount of 50 mg/kg, and the length of the ulcer formed was measured in the same manner.

[Results]

The results are shown in Table 2.

Table 2

| Length of ulcer formed | | |
|---|---|---|
| | Length of ulcer (mm) | |
| Piroxicam | Mean | 19.6 |
| alone | S.E. | 3.1 |
| Piroxicam-albumin | Mean | 6.5 |
| composition No. 1 | S.E. | 2.0 |
| Mean indicates an average value (n = 5). S.E. indicates a standard error. | | |

As is clear from Table 2, the Piroxicam-albumin composition No. 1 significantly inhibits peptic ulcer formation.

As described above in detail, the present invention provides a non-steroidal anti-inflmmatory agent composition comprising Piroxicam as an active ingredient, which has excellent Piroxicam absorbability and gives reduced gastroenteric disorder.

Claims

1. A non-steroidal pharmaceutical composition comprising Piroxicam and a natural albumin.

2. A composition according to Claim 1, wherein the natural albumin is selected from the group consisting of egg albumin, serum albumin, piakalbumin, $\alpha$-lactalbumin, leucosin, phaselin and Revmerine.

3. A composition according to Claim 1, wherein the proportion of Pyroxicam is 5-100 parts by weight based on 100 parts by weight of the natural albumin.

4. A composition according to Claim 1, further comprising a fatty oil and/or a fatty acid.

5. A composition according to Claim 4, wherein a weight ratio of a fatty oil and/or a fatty acid to the mixture of Piroxicam and a natural albumin is preferably 1/9 to 7/3, more preferably 2/8 to 6/4, most preferably 2.5/7.5 to 5.5/4.5.

6. A process for producing a non-steroidal anti-inflammatory agent composition, which process comprises stirring Piroxicam and a natural albumin at a high speed in the presence of an aqueous solvent and then removing the aqueous solvent to obtain a non-steroidal anti-inflammatory agent composition comprising Piroxicam and a natural albumin.

7. A process according to Claim 6, wherein the natural albumin is selected from the group consisting of egg albumin, serum albumin, plakalbumin, α-lactalbumin, leucosin, phaselin and Revmerine.

8. A process according to Claim 6, wherein the aqueous solvent is water or a mixture of water and a hydrophilic solvent.

9. A process according to Claim 8, wherein the hydrophilic solvent is at least one member selected from the group consisting of methanol, ethanol, acetone and ethyl acetate.

10. A process according to Claim 6, wherein stirring is effected at 5,000-40,000 rpm for 1-30 hours.

11. A process according to Claim 6, wherein Piroxicam is used by dissolving in a fatty oil and/or a fatty acid.

12. A process according to Claim 11, wherein the dissolution of Piroxicam in a fatty oil and/or a faty acid is effected in the presence of an organic solvent.

13. A process according to Claim 12, wherein the organic solvent is at least one member selected from the group consisting of acetone, methanol, ethanol, chloroform, ethyl acetate, ether, dichloromethane and THF.

14. The use of Piroxicam and natural albumin for the making of a medicament against inflammation.


Claims for the following Contracting States: ES GR

1. A process for producing a non-steroidal pharmaceutical composition, which process comprises stirring Piroxicam and a natural albumin in the presence of a solvent and then removing the solvent to obtain the pharmaceutical composition comprising Piroxicam and a natural albumin.

2. A process according to Claim 1, wherein the natural albumin is selected from the group consisting of egg albumin, serum albumin, plakalbumin, α-lactalbumin, leucosin, phaselin and Revmerine.

3. A process according to Claim 1, wherein an aqueous solvent is used which is preferably water or a mixture of water and a hydrophilic solvent.

4. A process according to Claim 3, wherein the hydrophilic solvent is at least one member selected from the group consisting of methanol, ethanol, acetone and ethyl acetate.

5. A process according to Claim 1, wherein stirring is at high speed,preferably at 5,000-40,000 rpm for 1-30 hours.

6. A process according to Claim 1, wherein Piroxicam is used by dissolving in a fatty oil and/or a fatty acid.

7. A process according to Claim 6, wherein the dissolution of Piroxicam in a fatty oil and/or a fatty acid is effected in the presence of an organic solvent.

8. A process according to Claim 7, wherein the organic solvent is at least one member selected from the group consisting of acetone, methanol, ethanol, chloroform, ethyl acetate, ether, dichloromethane and THF.

9. A method according to any of the preceding Claims , wherein the proportion of Pyroxicam is 5-100 parts by weight based on 100 parts by weight of the natural albumin.

10. A method according to Claim 6, wherein the weight ratio of fatty oil and/or fatty acid to the mixture of Piroxicam and natural albumin is preferably 1/9 to 7/3, more preferably 2/8 to 6/4, most preferably 2.5/7.5 to 5.5/4.5.

11. The use of Piroxicam and natural albumin for the making of a medicament against inflammation.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 806 457 (NIPPON HYPOX LABORATORIES INC.)<br>* Page 5, last paragraph; page 29; claims 1-9 * & EP-A-0 326 618<br>----- | 1-14 | A 61 K 47/00<br>A 61 K 37/02 //<br>(A 61 K 37/02<br>A 61 K 31:54 ) |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 13-12-1989 | BRINKMANN C. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................
& : member of the same patent family, corresponding
    document

EPO FORM 1503 03.82 (P0401)